# EUROPEAN PATENT APPLICATION

(11) **EP 4 813 300 A1**
(43) Date of publication of application: **30.09.2026**
(21) Application number: 24894076.9
(22) Date of filing: 14.11.2024
(51) Int. Cl.: A61B 6/00, A61B 6/08

(54) **X-RAY IMAGING DEVICE**

(30) Priority: 20.11.2023 JP 2023197024
(71) Applicant: SHIMADZU CORPORATION, Kyoto-shi, Kyoto 604-8511 (JP)
(72) Inventor: FURUHASHI, Naoya, Kyoto-shi, Kyoto 604-8511 (JP)
(74) Representative: Müller-Boré & Partner Patentanwälte PartG mbB
(86) International application number: PCT/JP2024/040486
(87) International publication number: WO 2025/110085

(57) **Abstract**

This X-ray imaging apparatus (100) comprises an optical imaging unit (30), and a control unit (32) generating an enlarged partial image (3), wherein the control unit (32) is configured to, based on a relationship between an optical axis (5) of the optical imaging unit (30) and an irradiation axis (6) of X-rays, set a center position (3a) of the enlarged partial image (3) to a position shifted from a center position (2a) of an optical image (2) in a direction approaching a position of an intersection (4) of an X-ray detection unit (20) in real space and the irradiation axis (6) of the X-rays irradiated from an X-ray irradiation unit (10).

## Description

### Technical Field

The present invention relates to an X-ray imaging apparatus.

### Background Art

Conventionally, an X-ray imaging apparatus including an optical imaging unit is known. Such an X-ray imaging apparatus is disclosed, for example, in JP 2023-104648 A.

The X-ray imaging apparatus described in JP 2023-104648 A includes an optical imaging unit provided on an outer surface of a collimator. In this X-ray imaging apparatus, the optical axis of the optical imaging unit and the irradiation axis of the X-rays are not coaxial. Then, this X-ray imaging apparatus determines whether a predetermined part of a subject is included in the imaging range in the X-ray image by inputting an input image based on an optical image including the subject captured by the optical imaging unit into a trained model and acquiring an output result.

### Prior Art Document

### Patent Document

Patent Document 1: JP 2023-104648 A

### Summary of the Invention

### Problems to be Solved by the Invention

However, in the X-ray imaging apparatus of JP 2023-104648 A, it is considered that in the optical image captured by the optical imaging unit, the positions of the subject and the X-ray detection unit are offset from the center of the optical image. Here, a radiographer adjusts the body orientation and position of the subject by referring to the optical image displayed on the display unit. At that time, when the distance between the X-ray irradiation unit and the X-ray detection unit is large, the subject is displayed small on the optical image, resulting in poor visibility, so it is useful to enlarge the optical image. In this case, when the radiographer adjusts the body orientation and position of the subject based on the enlarged partial image cropped with the center of the optical image as the enlargement center, there is a problem that the region of interest of the subject may not be displayed on the display unit because it protrudes outside the enlarged partial image. Therefore, it is desired to suppress the region of interest from not being displayed on the display unit due to the region of interest of the subject protruding outside the enlarged partial image.

The present invention has been made to solve the above problems, and one object of the present invention is to provide an X-ray imaging apparatus capable of suppressing a region of interest from not being displayed on a display unit due to the region of interest of a subject protruding outside an enlarged partial image.

### Means for Solving the Problems

An X-ray imaging apparatus according to one aspect of the present invention includes: an X-ray irradiation unit including an X-ray tube; an X-ray detection unit that detects X-rays irradiated from the X-ray irradiation unit and transmitted through a subject; an optical imaging unit that has an optical axis offset with respect to an irradiation axis of the X-rays irradiated from the X-ray irradiation unit and captures an optical image of the subject; a control unit that crops out a predetermined region from the optical image and generates an enlarged partial image that has been enlarged; and a display unit that displays the enlarged partial image generated by the control unit, wherein the control unit is configured to, based on a relationship between the optical axis of the optical imaging unit and the irradiation axis of the X-rays, set a center position of the enlarged partial image to a position shifted from a center position of the optical image in a direction approaching a position of an intersection of the X-ray detection unit in real space and the irradiation axis of the X-rays irradiated from the X-ray irradiation unit.

### Effects of the Invention

In the X-ray imaging apparatus according to the one aspect described above, as described above, the control unit is configured to, based on the relationship between the optical axis of the optical imaging unit and the irradiation axis of the X-rays, set the center position of the enlarged partial image to a position shifted from the center position of the optical image in a direction approaching the position of the intersection of the X-ray detection unit in real space and the irradiation axis of the X-rays irradiated from the X-ray irradiation unit. As a result, since the center position of the enlarged partial image can be brought closer to the intersection of the X-ray detection unit and the irradiation axis of the X-rays, the X-ray detector and the subject can be displayed closer to the center in the enlarged partial image compared to the case where the center position of the enlarged partial image is the center position of the optical image. Therefore, it is possible to suppress the region of interest from not being displayed on the display unit due to the region of interest of the subject protruding outside the enlarged partial image.

### Brief Description of the Drawings

[FIG. 1] FIG. 1 is a schematic diagram showing an overall configuration of an X-ray imaging apparatus according to an embodiment.
[FIG. 2] FIG. 2 is a block diagram showing the overall configuration of the X-ray imaging apparatus according to the embodiment.
[FIG. 3] FIG. 3 is a schematic diagram showing a configuration of a holding unit according to the embodiment.
[FIG. 4] FIG. 4 is a schematic diagram showing an enlarged partial image displayed on a display operation unit.
[FIG. 5] FIG. 5 is a schematic diagram showing a re-enlarged partial image and a reduced enlarged-partial image displayed on the display operation unit.
[FIG. 6] FIG. 6 is a schematic diagram for explaining acquisition of a center position of the enlarged partial image by an optical imaging control unit.
[FIG. 7] FIG. 7 is a schematic diagram showing the optical image and the enlarged partial image in a first display form displayed on the display operation unit.
[FIG. 8] FIG. 8 is a schematic diagram for explaining adjustment of an enlargement ratio for generating the enlarged partial image by the optical imaging control unit.
[FIG. 9] FIG. 9 is a schematic diagram showing the optical image and the enlarged partial image in a second display form displayed on the display operation unit.

### Mode for Carrying Out the Invention

Hereinafter, embodiments embodying the present invention will be described with reference to the drawings.

### (Configuration of X-ray Imaging Apparatus)

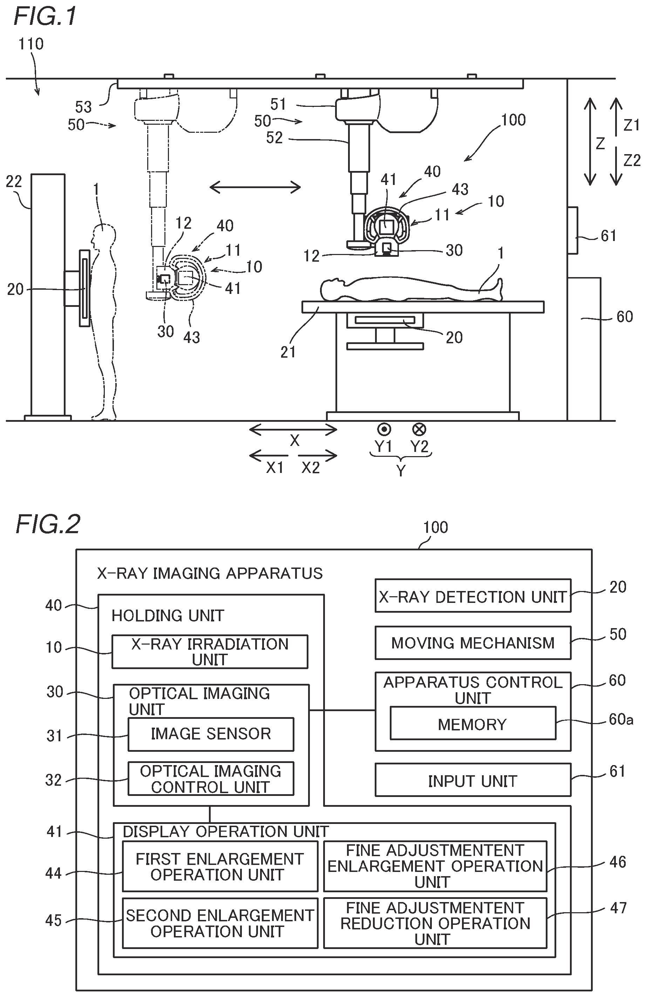

The configuration of an X-ray imaging apparatus 100 according to an embodiment of the present invention will be described with reference to FIGS. 1 to 3.

FIG. 1 shows an example of a ceiling-suspended X-ray imaging apparatus 100 installed in an imaging room 110. The X-ray imaging apparatus 100 includes an X-ray irradiation unit 10, an X-ray detection unit 20, an optical imaging unit 30, a display operation unit 41, a holding unit 40, a moving mechanism 50, an apparatus control unit 60, and an input unit 61. In the ceiling-suspended X-ray imaging apparatus 100, the holding unit 40 provided with the X-ray irradiation unit 10 and the optical imaging unit 30 is held by the moving mechanism 50 disposed on the ceiling of the imaging room 110 so as to be suspended from the ceiling. The holding unit 40 is movably held within the imaging room 110 by the moving mechanism 50. Here, a vertical (plumb) direction is defined as a Z direction, and two horizontal directions orthogonal to each other are defined as an X direction and a Y direction. The display operation unit 41 is an example of a "display unit" and an "operation unit" in the claims.

The X-ray imaging apparatus 100 is a medical X-ray imaging apparatus, and is configured to perform X-ray imaging of a subject 1, which is an imaging target. The X-ray imaging apparatus 100 includes an imaging table 21 for performing imaging in a posture in which the subject 1 is laid down (decubitus posture), and an imaging stand 22 for performing imaging in a posture in which the subject 1 stands up (standing posture).

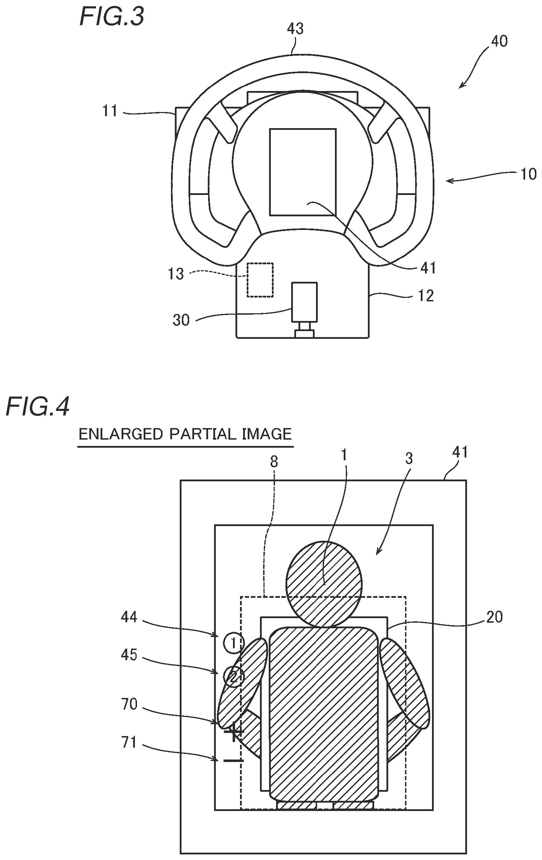

The X-ray irradiation unit 10 includes an X-ray tube 11, a collimator unit 12, and an irradiation field lamp 13 (see FIG. 3). The X-ray tube 11 is configured to irradiate the subject 1 with X-rays. The X-ray tube 11 is configured to irradiate X-rays when a predetermined voltage is applied. The collimator unit 12 has a plurality of position-adjustable shielding plates (collimator leaves). The collimator unit 12 is configured to adjust an irradiation field of the X-rays irradiated from the X-ray tube 11 by shielding a part of the X-rays from the X-ray tube 11. The collimator unit 12 is provided in the vicinity of the X-ray tube 11 in an X-ray irradiation direction of the X-ray tube 11. The collimator unit 12 is provided with the irradiation field lamp 13. The irradiation field lamp 13 includes a visible light source. The X-ray irradiation field can be confirmed using the visible light irradiated from the irradiation field lamp 13 without using X-rays.

The X-ray detection unit 20 is movably held on each of the imaging table 21 and the imaging stand 22. The X-ray detection unit 20 includes, for example, a flat panel detector (FPD). The X-ray detection unit 20 is configured to detect X-rays transmitted through the subject 1. The moving mechanism 50 is capable of moving the holding unit 40 at least between an imaging position in the decubitus posture using the imaging table 21 (see the solid line in FIG. 1) and an imaging position in the standing posture using the imaging stand 22 (see the two-dot chain line in FIG. 1).

In decubitus X-ray imaging, the holding unit 40 is disposed at a position vertically facing the X-ray detection unit 20 of the imaging table 21, and the subject 1 lying on the imaging table 21 is imaged between the vertically facing X-ray irradiation unit 10 and X-ray detection unit 20. In standing imaging, the holding unit 40 is disposed at a position horizontally facing the X-ray detection unit 20 of the imaging stand 22, and the subject 1 standing in front of the imaging stand 22 is imaged between the horizontally facing X-ray irradiation unit 10 and X-ray detection unit 20.

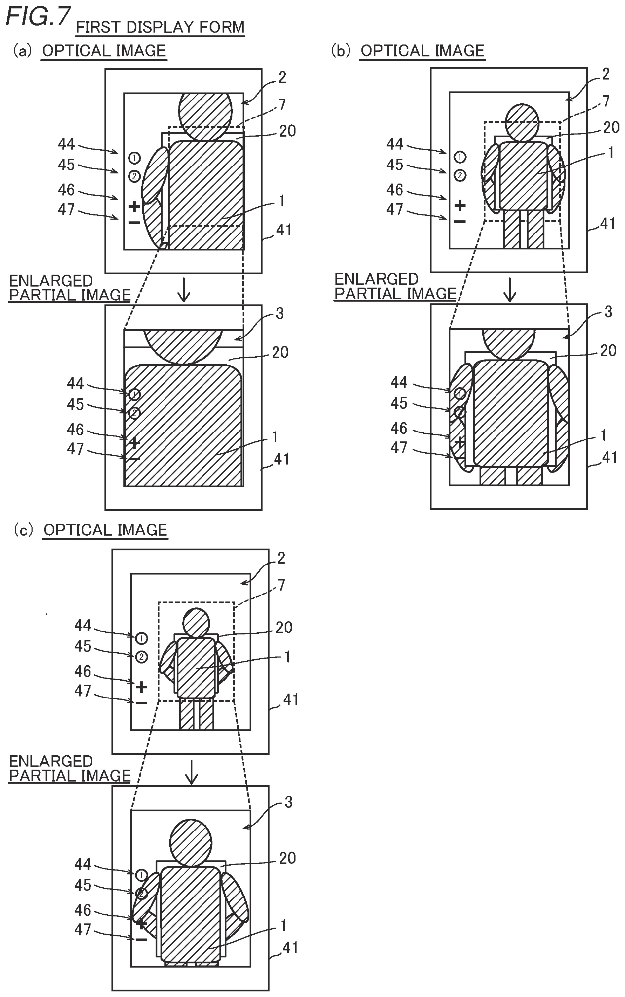

The optical imaging unit 30 is configured to capture an optical image 2 (see FIG. 7). The optical imaging unit 30 is, for example, an optical camera.

In decubitus X-ray imaging, the holding unit 40 is disposed at a position vertically facing the X-ray detection unit 20 of the imaging table 21, and the subject 1 lying on the imaging table 21 is captured by the optical imaging unit 30 between the vertically facing X-ray irradiation unit 10 and X-ray detection unit 20. In standing X-ray imaging, the holding unit 40 is disposed at a position horizontally facing the X-ray detection unit 20 of the imaging stand 22, and the subject 1 standing in front of the imaging stand 22 is captured by the optical imaging unit 30 between the horizontally facing X-ray irradiation unit 10 and X-ray detection unit 20.

As shown in FIG. 1, the optical imaging unit 30 is provided on an outer surface of the collimator unit 12. In the present embodiment, the optical imaging unit 30 is provided on the outer surface on a longitudinal direction side of the imaging table 21 in the collimator unit 12 in the decubitus imaging position. Further, the optical imaging unit 30 is provided on the outer surface on a side surface side intersecting an detection surface of the X-ray detection unit 20 in the collimator unit 12 in the standing imaging position. The optical imaging unit 30 is provided to face an irradiation direction of the X-rays from the X-ray irradiation unit 10.

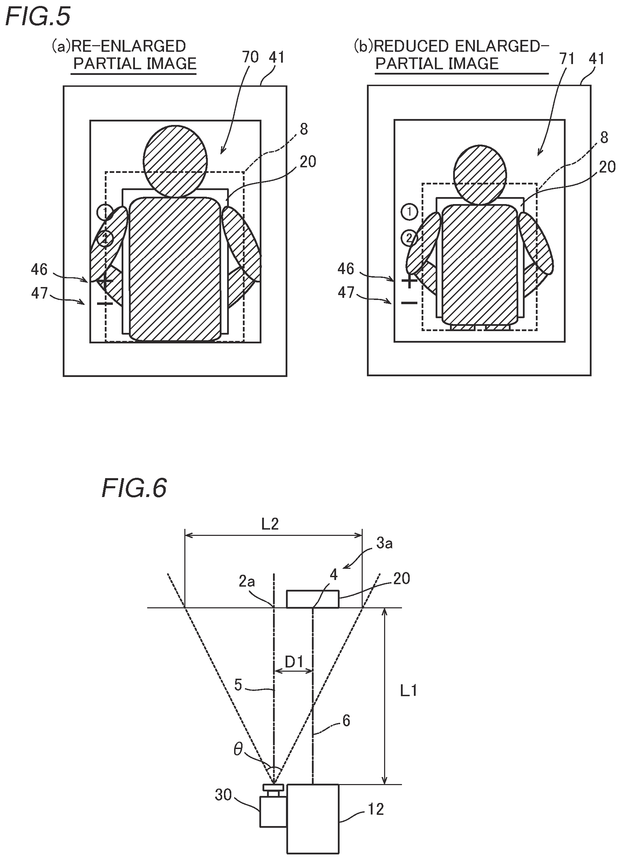

As shown in FIG. 6, the optical imaging unit 30 is configured to have an optical axis 5 offset with respect to an irradiation axis 6 of the X-rays irradiated from the X-ray irradiation unit 10. In other words, the optical imaging unit 30 is configured to have an optical axis 5 that is not coaxial with the irradiation axis 6 of the X-rays irradiated from the X-ray irradiation unit 10. In addition, the optical imaging unit 30 is configured to have an optical axis 5 substantially parallel to the irradiation axis 6 of the X-rays irradiated from the X-ray irradiation unit 10. The optical imaging unit 30 can capture the optical image 2 of the subject 1 and the X-ray detection unit 20 from the X-ray irradiation unit 10 side when the X-ray irradiation unit 10 faces a direction of the subject 1 and the X-ray detection unit 20. An imaging range of the optical imaging unit 30 is set to include a range of the X-ray irradiation field and to be larger than the range of the X-ray irradiation field.

The optical imaging unit 30 can capture an optical image 2 including a visible light region 8 (see FIG. 4) formed by visible light irradiated from the irradiation field lamp 13 (see FIG. 3) and a peripheral portion of the visible light region 8. The range of the visible light region 8 formed by the visible light irradiated from the irradiation field lamp 13 substantially matches the range of the X-ray irradiation field. Although details will be described later, an enlarged partial image 3 (see FIG. 4) obtained by cropping and enlarging a predetermined region 7 from the optical image 2 is displayed on the display operation unit 41. By using the visible light irradiated from the irradiation field lamp 13, the X-ray irradiation field can be confirmed based on the enlarged partial image 3 without irradiating X-rays.

As shown in FIG. 2, the optical imaging unit 30 includes an image sensor 31 and an optical imaging control unit 32. The optical imaging unit 30 is, for example, an optical camera. The optical imaging control unit 32 is an example of a "control unit" in the claims.

The image sensor 31 includes, for example, a CCD (Charge Coupled Device) image sensor or a CMOS (Complementary Metal Oxide Semiconductor) image sensor.

The optical imaging control unit 32 is a microcomputer including, for example, a CPU (Central Processing Unit) and a memory. The optical imaging control unit 32 is configured to receive a signal output from the image sensor 31 and to generate the optical image 2 based on the input signal. Furthermore, the optical imaging control unit 32 is configured to crop a predetermined region 7 from the generated optical image 2 and generate the enlarged partial image 3 that is enlarged.

Based on a relationship between the optical axis 5 of the optical imaging unit 30 and the irradiation axis 6 of the X-rays irradiated from the X-ray irradiation unit 10, the optical imaging control unit 32 is configured to set a center position 3a of the enlarged partial image 3 to a position shifted from a center position 2a of the optical image 2 in a direction approaching a position of an intersection 4 of the X-ray detection unit 20 in real space and the irradiation axis 6 of the X-rays irradiated from the X-ray irradiation unit 10. Here, the direction approaching the position of the intersection 4 is a direction in which the optical imaging unit 30 and the X-ray irradiation unit 10 are aligned. The control for acquiring the center position 3a of the enlarged partial image 3 by the optical imaging control unit 32 will be described later.

Furthermore, as a first display form of the enlarged partial image 3, the optical imaging control unit 32 is configured to cause the display operation unit 41 to display the range of the predetermined region 7 in the optical image 2 as the enlarged partial image 3 based on a preset enlargement ratio. The control of the first display form of the enlarged partial image 3 by the optical imaging control unit 32 will be described later.

Furthermore, as a second display form of the enlarged partial image 3, the optical imaging control unit 32 is configured to adjust an enlargement ratio for generating the enlarged partial image 3 to thereby cause the display operation unit 41 to display the entire range of the predetermined region 7 preset based on a size of the X-ray detection unit 20 in real space as the enlarged partial image 3. The control of the second display form of the enlarged partial image 3 by the optical imaging control unit 32 will be described later.

Also, based on an input operation to the display operation unit 41, the optical imaging control unit 32 is configured to generate a re-enlarged partial image 70 (see FIG. 5(a)) obtained by further enlarging the enlarged partial image 3 displayed on the display operation unit 41 or a reduced enlarged-partial image 71 (see FIG. 5(b)) obtained by reducing the enlarged partial image 3 displayed on the display operation unit 41, and to display it on the display operation unit 41.

As shown in FIG. 1, the holding unit 40 holds the X-ray irradiation unit 10. As shown in FIG. 4, the holding unit 40 includes the X-ray irradiation unit 10, the optical imaging unit 30, the display operation unit 41, and a grip unit 43. The holding unit 40 is configured to be movable in the horizontal direction and the vertical direction via the moving mechanism 50 by manual movement or control by the apparatus control unit 60.

The display operation unit 41 includes, for example, a touch panel type liquid crystal display. The display operation unit 41 is configured to function as an image display unit that displays the optical image 2 captured by the optical imaging unit 30 (see FIG. 7), the enlarged partial image 3 generated by the optical imaging control unit 32 (see FIG. 4), imaging information, and the like, and also to function as an operation unit into which various operations by the radiographer are input.

The display operation unit 41 includes an operation unit having a first enlargement operation unit 44, a second enlargement operation unit 45, a fine adjustment enlargement operation unit 46, and a fine adjustment reduction operation unit 47. Note that the fine adjustment enlargement operation unit 46 and the fine adjustment reduction operation unit 47 are an example of an "operation unit" in the claims.

The first enlargement operation unit 44 is configured to receive an operation input for causing the display operation unit 41 to display the enlarged partial image 3 in the first display form. The second enlargement operation unit 45 is configured to receive an operation input for causing the display operation unit 41 to display the enlarged partial image 3 in the second display form.

The fine adjustment enlargement operation unit 46 and the fine adjustment reduction operation unit 47 are configured to receive an input operation for finely adjusting an enlargement ratio with respect to the enlarged partial image 3. Specifically, the fine adjustment enlargement operation unit 46 is configured to receive an operation input for causing the display operation unit 41 to display the re-enlarged partial image 70 (see FIG. 5(a)) obtained by enlarging the enlarged partial image 3 (see FIG. 4). In addition, the fine adjustment reduction operation unit 47 is configured to receive an operation input for causing the display operation unit 41 to display the reduced enlarged-partial image 71 (see FIG. 5(b)) obtained by reducing the enlarged partial image 3 (see FIG. 4).

The grip unit 43 is provided for an operator to grip when performing an operation of manually moving the holding unit 40. The grip unit 43 transmits an operating force of the operator to the holding unit 40.

As shown in FIG. 1, the moving mechanism 50 is configured to movably hold the holding unit 40 in a horizontal direction (X direction and Y direction) and a vertical direction (Z direction). The moving mechanism 50 includes a ceiling suspender 51 and a column unit 52. The moving mechanism 50 is supported by rails 53 provided on the ceiling of the imaging room 110. The ceiling suspender 51 is configured to be movable in the horizontal direction along the rails 53. The ceiling suspender 51 is configured to support the column unit 52. The column unit 52 is configured to support the holding unit 40. The column unit 52 is configured to be extendable and contractible in the vertical direction. The holding unit 40 is configured to be movable in the vertical direction by the column unit 52.

The apparatus control unit 60 performs control related to X-ray imaging by the X-ray irradiation unit 10 and the X-ray detection unit 20 and control related to movement of the holding unit 40. The apparatus control unit 60 includes a CPU (Central Processing Unit) and a memory 60a.

The input unit 61 has a function of receiving an input operation relating to X-ray imaging. The input operation includes setting of imaging conditions for X-ray imaging, an instruction to start X-ray irradiation, and the like.

### (Control of Acquisition of Center Position of Enlarged Partial Image by Optical Imaging Control Unit)

Control of acquisition of the center position 3a of the enlarged partial image 3 by the optical imaging control unit 32 will be described with reference to FIG. 6.

When an input operation to either the first enlargement operation unit 44 or the second enlargement operation unit 45 in the display operation unit 41 and an input operation of X-ray imaging conditions such as an imaging region and an operative method in the display operation unit 41 are accepted, the optical imaging control unit 32 crops out the predetermined region 7 from the optical image 2 and generates the enlarged partial image 3 that is enlarged. In generating the enlarged partial image 3, the optical imaging control unit 32 sets the center position 3a of the enlarged partial image 3 to a position shifted from the center position 2a of the optical image 2 in a direction approaching the position of the intersection 4 of the X-ray detection unit in real space and the irradiation axis 6 of the X-rays irradiated from the X-ray irradiation unit.

Based on the relationship between the optical axis 5 of the optical imaging unit 30 and the irradiation axis 6 of the X-rays irradiated from the X-ray irradiation unit 10, the optical imaging control unit 32 sets the center position 3a of the enlarged partial image 3 to a position shifted from the center position 2a of the optical image 2 in the direction approaching the position of the intersection 4 of the X-ray detection unit in real space and the irradiation axis 6 of the X-rays irradiated from the X-ray irradiation unit. In the present embodiment, the optical imaging control unit 32 sets the center position 3a of the enlarged partial image 3 to a position substantially matching the position of the intersection 4 of the X-ray detection unit 20 in real space and the irradiation axis 6 of the X-rays irradiated from the X-ray irradiation unit 10, from the center position 2a of the optical image 2.

Examples of the relationship between the optical axis 5 of the optical imaging unit 30 and the irradiation axis 6 of the X-rays irradiated from the X-ray irradiation unit 10 include a distance L1 between the optical imaging unit 30 and the X-ray detection unit 20, a distance D1 between the irradiation axis 6 of the X-rays and the optical axis 5 of the optical imaging unit 30, and an angle of view *θ* of the optical imaging unit 30.

The distance L1 between the optical imaging unit 30 and the X-ray detection unit 20 is preset for each imaging region, and is linked to the imaging region and stored in the memory 60a of the apparatus control unit 60. Further, the distance D1 between the irradiation axis 6 of the X-rays and the optical axis 5 of the optical imaging unit 30, and the angle of view *θ* of the optical imaging unit 30 are stored in advance in the memory 60a of the apparatus control unit 60. The optical imaging control unit 32 acquires the distance L1 between the optical imaging unit 30 and the X-ray detection unit 20, the distance D1 between the irradiation axis 6 of the X-rays and the optical axis 5 of the optical imaging unit 30, and the angle of view *θ* of the optical imaging unit 30, which are stored in the memory 60a. Note that the optical imaging control unit 32 may be configured to acquire, from the apparatus control unit 60, the distance L1 between the optical imaging unit 30 and the X-ray detection unit 20 acquired by the apparatus control unit 60 based on a detected three-dimensional positional relationship between the optical imaging unit 30 and the X-ray detection unit 20.

Then, as shown in FIG. 6, based on the distance L1 between the optical imaging unit 30 and the X-ray detection unit 20, the distance D1 between the irradiation axis 6 of the X-rays and the optical axis 5 of the optical imaging unit 30, and the angle of view *θ* of the optical imaging unit 30, the optical imaging control unit 32 acquires a center position shift rate S in order to set the center position 3a of the enlarged partial image 3 from the center position 2a of the optical image 2 to a position substantially matching the position of the intersection 4 of the X-ray detection unit 20 in real space and the irradiation axis 6 of the X-rays irradiated from the X-ray irradiation unit 10. The "center position shift rate S" is a ratio of the distance D1 between the irradiation axis 6 of the X-rays and the optical axis 5 of the optical imaging unit 30 to a length L2 in real space of the imaging range included in the angle of view *θ* in a plane direction of the X-ray detection unit 20 including the intersection 4 of the X-ray detection unit 20 and the irradiation axis 6 of the X-rays irradiated from the X-ray irradiation unit 10. The center position shift rate S is expressed by the following equation (1).
[Equation 1] $S = \frac{D 1}{2 ⋅ L 1 ⋅ tan \frac{θ}{2}}$

Then, the optical imaging control unit 32 acquires the center position 3a of the enlarged partial image 3 based on the acquired center position shift rate S. Specifically, a position shifted from the center position 2a of the optical image 2 in the direction approaching the position of the intersection 4 by a number of pixels obtained by multiplying the number of pixels in the direction approaching the position of the intersection 4 in the optical image 2 by the acquired center position shift rate S is acquired as the center position 3a of the enlarged partial image 3. Thus, the acquired center position 3a of the enlarged partial image 3 is a position that substantially matches the position of the intersection 4.

As an example, when the number of pixels in the direction approaching the position of the intersection 4 in the optical image 2 is 960 and the number of pixels in a direction orthogonal to the direction approaching the position of the intersection 4 in the optical image 2 is 1280, a position shifted from the center position 2a of the optical image 2 in the direction approaching the position of the intersection 4 by a number of pixels obtained by multiplying the number of pixels 960 in the direction approaching the position of the intersection 4 in the optical image 2 by the center position shift rate S is acquired as the center position 3a of the enlarged partial image 3.

Then, based on the acquired center position 3a of the enlarged partial image 3, the optical imaging control unit 32 generates the enlarged partial image 3 obtained by cropping the predetermined region 7 from the optical image and enlarging the predetermined region 7.

### (Control of First Display Form of Enlarged Partial Image by Optical Imaging Control Unit)

The control of the first display form of the enlarged partial image 3 by the optical imaging control unit 32 will be described with reference to FIGS. 7(a) to 7(c). Here, the control of the first display form of the enlarged partial image 3 by the optical imaging control unit 32 in the case of performing X-ray imaging in a posture in which the subject 1 stands up (standing posture) will be described. In FIGS. 7(a) to 7(c), for convenience of explanation, the range of the predetermined region 7 is shown by broken lines. Note that the above control in a case where X-ray imaging is performed in a posture in which the subject 1 is laid down on the imaging table 21 (decubitus posture) is also similar control, and therefore description thereof is omitted.

When the input operation of the first enlargement operation unit 44 in the display operation unit 41 and the input operation of X-ray imaging conditions such as an imaging region and an operative method in the display operation unit 41 are accepted, the optical imaging control unit 32 causes the display operation unit 41 to display the range of the predetermined region 7 in the optical image 2 as the enlarged partial image 3 based on the acquired center position 3a of the enlarged partial image 3 and a preset enlargement ratio. The optical imaging control unit 32 switches the optical image 2 displayed on the display operation unit 41 to the enlarged partial image 3 and causes the display operation unit 41 to display it.

The enlargement ratio of the range of the predetermined region 7 in the optical image 2 is preset for each imaging region, and is stored in the memory 60a of the apparatus control unit 60 in association with the imaging region. The optical imaging control unit 32 acquires the enlargement ratio corresponding to the imaging region stored in the memory 60a. As an example, when the imaging region is the chest, the enlargement ratio of the range of the predetermined region 7 in the optical image 2 is 2.0 times. As another example, when the imaging region is the head, the enlargement ratio of the range of the predetermined region 7 in the optical image 2 is 1.5 times. The enlargement ratio of the range of the predetermined region 7 in the optical image 2 may be set by the radiographer, the enlargement ratio set by the radiographer may be changed and reset, or may be set at the time of factory shipment.

Here, an aspect ratio of the range of the predetermined region 7 in the optical image 2 is configured to match an aspect ratio of an image display unit of the display operation unit 41. Therefore, the range of the predetermined region 7 in the optical image 2 is uniquely acquired based on the acquired center position 3a of the enlarged partial image 3 and the preset enlargement ratio.

FIGS. 7(a) to 7(c) are schematic diagrams showing the optical image 2 displayed on the display operation unit 41 and the enlarged partial image 3 displayed on the display operation unit 41 when the imaging region is the chest. In all of FIGS. 7(a) to 7(c), the preset enlargement ratio of the range of the predetermined region 7 in the optical image 2 is 2.0 times. In FIG. 7(a), the distance L1 between the optical imaging unit 30 and the X-ray detection unit 20 is 100 cm. In FIG. 7(b), the distance L1 between the optical imaging unit 30 and the X-ray detection unit 20 is 150 cm. In FIG. 7(c), the distance L1 between the optical imaging unit 30 and the X-ray detection unit 20 is 200 cm.

As shown in FIGS. 7(a) to 7(c), when the distance L1 between the optical imaging unit 30 and the X-ray detection unit 20 is different, the position of the range of the predetermined region 7 in the optical image 2 is also different. That is, if the distance L1 between the optical imaging unit 30 and the X-ray detection unit 20 is different, a position of a region cropped as the enlarged partial image 3 in the optical image 2 is also different. Further, as the distance L1 between the optical imaging unit 30 and the X-ray detection unit 20 is smaller, a distance between the center position 2a of the optical image 2 and the position of the intersection 4 between the X-ray detection unit 20 in real space and the irradiation axis 6 of the X-rays irradiated from the X-ray irradiation unit 10 is greater. The enlarged partial image 3 generated by the optical imaging control unit 32 and displayed on the display operation unit 41 is an image obtained by cropping the predetermined region 7 from the optical image 2 and enlarging the predetermined region 7 using the acquired center position 3a of the enlarged partial image 3 as a cropping center and an enlargement center, and the center position 3a of the enlarged partial image 3 is at a position substantially matching the position of the intersection 4 of the X-ray detection unit 20 in real space and the irradiation axis 6 of the X-rays irradiated from the X-ray irradiation unit 10.

### (Control of Second Display Form of Enlarged Partial Image by Optical Imaging Control Unit)

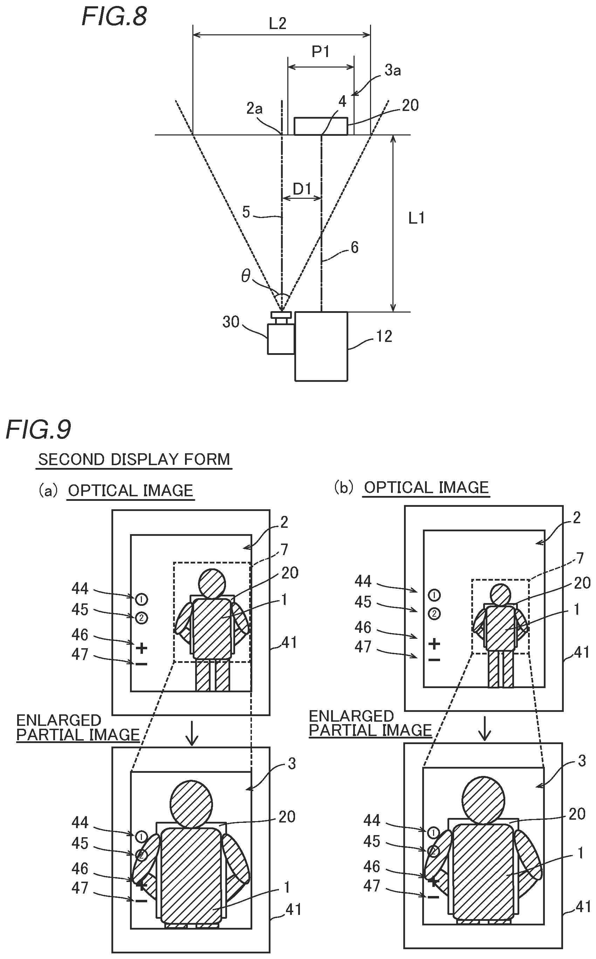

Control of the second display form of the enlarged partial image 3 by the optical imaging control unit 32 will be described with reference to FIG. 8 and FIGS. 9(a) to 9(b). Here, the control of the second display form of the enlarged partial image 3 by the optical imaging control unit 32 in a case where X-ray imaging is performed in a posture in which the subject 1 stands up (standing posture) will be described. Note that, in FIGS. 9(a) to 9(c), the range of the predetermined region 7 is indicated by broken lines for convenience of explanation. Further, since the above-described control in a case where X-ray imaging is performed in a posture in which the subject 1 lies down on the imaging table 21 (decubitus posture) is also a similar control, description thereof is omitted.

When the input operation of the second enlargement operation unit 45 in the display operation unit 41 and the input operation of X-ray imaging conditions such as an imaging region and an operative method in the display operation unit 41 are accepted, the optical imaging control unit 32 causes the display operation unit 41 to display the entire range of the predetermined region 7 in the optical image 2 as the enlarged partial image 3 based on the acquired center position 3a of the enlarged partial image 3 and a range of the predetermined region 7 set in advance based on the size of the X-ray detection unit 20 in real space. The optical imaging control unit 32 switches the optical image 2 displayed on the display operation unit 41 to the enlarged partial image 3 and causes the display operation unit 41 to display it.

The range of the predetermined region 7 in the optical image 2 is preset by the radiographer for each imaging region based on the size of the X-ray detection unit in real space, and is stored in the memory 60a of the apparatus control unit 60 in association with the imaging region. The optical imaging control unit 32 acquires the range of the predetermined region 7 corresponding to the imaging region stored in the memory 60a.

As an example shown in FIG. 8, in a case of the optical image 2 when the imaging region is the chest, a range between a position separated by 10 cm in the leftward direction from the left side surface of the X-ray detection unit 20 in real space and a position separated by 10 cm in the rightward direction from the right side surface of the X-ray detection unit 20 in real space can be preset as a left-right direction range P1 within the range of the predetermined region 7. Here, an aspect ratio of the range of the predetermined region 7 in the optical image 2 is configured to match an aspect ratio of an image display unit of the display operation unit 41. Therefore, the range of the predetermined region 7 in the optical image 2 is uniquely acquired based on the acquired center position 3a of the enlarged partial image 3 and the set left-right direction range P1 in the range of the predetermined region 7 of the optical image 2.

Then, the optical imaging control unit 32 adjusts the enlargement ratio for generating the enlarged partial image 3. Specifically, as an example, the optical imaging control unit 32 acquires an adjusted enlargement ratio M for generating the enlarged partial image 3 based on the distance L1 between the optical imaging unit 30 and the X-ray detection unit 20, the angle of view *θ* of the optical imaging unit 30, and the left-right direction range P1 in the range of the predetermined region 7 preset based on the size of the X-ray detection unit 20 in real space. The "adjusted enlargement ratio M" is a reciprocal of a ratio of a length of the left-right direction range P1 in the range of the predetermined region 7 preset based on the size of the X-ray detection unit 20 in real space to a length L2 on real space of an imaging range included in an angle of view *θ* in a plane direction of the X-ray detection unit 20 including the intersection 4 between the X-ray detection unit 20 and the irradiation axis 6 of the X-rays irradiated from the X-ray irradiation unit 10. The adjusted enlargement ratio M is expressed by the following equation (2).
[Equation 2] $M = \frac{1}{\frac{P 1}{2 ⋅ L 1 ⋅ tan \frac{θ}{2}}}$

Then, the optical imaging control unit 32 causes the display operation unit 41 to display the entire range of the predetermined region 7 in the optical image 2 as the enlarged partial image 3 based on the acquired adjusted enlargement ratio M, the acquired center position 3a of the enlarged partial image 3, and the range of the predetermined region 7 preset based on the size of the X-ray detection unit 20 in real space.

FIGS. 9(a) to 9(c) are schematic diagrams showing the optical image 2 displayed on the display operation unit 41 and the enlarged partial image 3 displayed on the display operation unit 41 when the imaging region is the chest. In any of FIGS. 9(a) to 9(c), the preset left-right direction range within the range of the predetermined region 7 is a range between a position 10 cm away to the left from a left side surface of the X-ray detection unit 20 in real space and a position 10 cm away to the right from a right side surface of the X-ray detection unit 20 in real space. In the optical image 2, the range of the predetermined region 7 is indicated by broken lines for convenience of explanation.

As shown in FIGS. 9(a) to 9(c), even if the distance L1 between the optical imaging unit 30 and the X-ray detection unit 20 is different, regions cut out as the enlarged partial image 3 in the optical image 2 are substantially the same. That is, even if the distance L1 between the optical imaging unit 30 and the X-ray detection unit 20 is different, the range of the enlarged partial image 3 displayed on the display operation unit 41 is substantially the same. Furthermore, the enlarged partial image 3 generated by the optical imaging control unit 32 and displayed on the display unit is an image obtained by cropping the preset predetermined region 7 from the optical image 2 and enlarging the preset predetermined region 7 using the acquired center position 3a of the enlarged partial image 3 as a cropping center and an enlargement center, and the center position 3a of the enlarged partial image 3 is at a position substantially matching the position of the intersection 4 of the X-ray detection unit 20 in real space and the irradiation axis 6 of the X-rays irradiated from the X-ray irradiation unit 10.

### (Re-enlarged Partial Image and Reduced Enlarged-Partial Image)

As shown in FIG. 5(a), based on an input operation to the fine adjustment enlargement operation unit 46 (see FIG. 2) of the display operation unit 41, the optical imaging control unit 32 generates a re-enlarged partial image 70 obtained by further enlarging the enlarged partial image 3 displayed on the display operation unit 41, and displays the generated re-enlarged partial image 70 on the display operation unit 41.

Further, as shown in FIG. 5(b), based on an input operation to the fine adjustment reduction operation unit 47 (see FIG. 2) of the display operation unit 41, the optical imaging control unit 32 generates a reduced enlarged-partial image 71 obtained by reducing the enlarged partial image 3 displayed on the display operation unit 41, and displays the generated reduced enlarged-partial image 71 on the display operation unit 41.

### (Effects of the Present Embodiment)

In the present embodiment, the following effects can be obtained.

In the present embodiment, as described above, the optical imaging control unit 32 is configured to, based on the relationship between the optical axis 5 of the optical imaging unit 30 and the irradiation axis 6 of the X-rays, set the center position 3a of the enlarged partial image 3 to a position shifted from the center position 2a of the optical image 2 in a direction approaching the position of the intersection 4 of the X-ray detection unit 20 in real space and the irradiation axis 6 of the X-rays irradiated from the X-ray irradiation unit 10. As a result, the center position 3a of the enlarged partial image 3 can be brought closer to the intersection 4 of the X-ray detection unit 20 and the irradiation axis 6 of the X-rays, so that the X-ray detection unit 20 and the subject 1 can be displayed closer to the center in the enlarged partial image 3 compared to the case where the center position 3a of the enlarged partial image 3 is the center position 2a of the optical image 2. Therefore, it is possible to suppress the region of interest from not being displayed on the display operation unit 41 due to the region of interest of the subject 1 protruding outside the enlarged partial image 3.

In addition, in the present embodiment, the following configuration provides the following further effects.

That is, in the present embodiment, as described above, the optical imaging control unit 32 is configured to set the center position 3a of the enlarged partial image 3 to a position shifted in a direction approaching the position of the intersection 4, based on the distance L1 between the optical imaging unit 30 and the X-ray detection unit 20, the distance D1 between the irradiation axis 6 of the X-rays and the optical axis 5 of the optical imaging unit 30, and the angle of view *θ* of the optical imaging unit 30. Accordingly, even if the optical imaging unit 30 has an optical axis deviated with respect to the irradiation axis 6 of the X-rays irradiated from the X-ray irradiation unit 10, the center position 3a of the enlarged partial image 3 can be easily acquired based on the distance L1 between the optical imaging unit 30 and the X-ray detection unit 20, the distance D1 between the irradiation axis 6 of the X-rays and the optical axis 5 of the optical imaging unit 30, and the angle of view *θ* of the optical imaging unit 30. Therefore, the X-ray detection unit 20 and the subject 1 can be easily displayed closer to the center in the enlarged partial image 3. As a result, it is possible to easily suppress a region of interest from not being displayed on the display operation unit 41 due to the region of interest of the subject 1 protruding to the outside of the enlarged partial image 3.

In addition, in the present embodiment, as described above, the optical imaging unit 30 is configured to have an optical axis 5 substantially parallel to the irradiation axis 6 of the X-rays irradiated from the X-ray irradiation unit 10. Accordingly, it is possible to suppress the subject 1 from being displayed in a distorted manner in the optical image 2 due to an inclination of the optical axis 5 of the optical imaging control unit 32 with respect to the irradiation axis 6 of the X-rays irradiated from the X-ray irradiation unit 10, so that it is possible to suppress the subject 1 from being displayed in a distorted manner also in the enlarged partial image 3.

In addition, in the present embodiment, as described above, the optical imaging control unit 32 is configured to cause the display operation unit 41 to display a range of a predetermined region 7 in the optical image 2 as an enlarged partial image 3 based on a preset enlargement ratio. This makes it possible for the radiographer to easily adjust the orientation and position of the body of the subject 1 on the basis of the enlarged partial image 3 in which the optical image 2 is enlarged at a preset desired enlargement ratio.

In addition, in the present embodiment, as described above, the optical imaging control unit 32 is configured to cause the display operation unit 41 to display a range of a predetermined region 7 in the optical image 2 as an enlarged partial image 3 based on an enlargement ratio preset for each imaging region. Here, in adjusting the body orientation and position of the subject 1, when the imaging region is the head, the set distance L1 between the optical imaging unit 30 and the X-ray detection unit 20 is small (short), so the enlargement ratio of the enlarged partial image 3 is preferably small; but when the imaging region is the chest, the set distance L1 between the optical imaging unit 30 and the X-ray detection unit 20 is large (long), so the enlargement ratio of the enlarged partial image 3 is preferably large. Therefore, since the enlarged partial image 3 is displayed on the display operation unit 41 based on the enlargement ratio set in advance for each imaging region, the radiographer can easily adjust the body direction and position of the subject 1 based on the enlarged partial image 3 having the enlargement ratio suitable for the imaging region.

Furthermore, in the present embodiment, as described above, the optical imaging control unit 32 is configured to adjust an enlargement ratio for generating the enlarged partial image 3, thereby displaying the entire range of a predetermined region 7 preset based on a size of the X-ray detection unit 20 in real space on the display operation unit 41 as the enlarged partial image 3. Thus, since the range of the predetermined region 7 is set in advance based on the size of the X-ray detection unit 20 in real space, the display operation unit 41 can display the enlarged partial image 3 having a substantially identical range regardless of the distance L1 between the optical imaging unit 30 and the X-ray detection unit 20. Therefore, the radiographer can more easily adjust the orientation and position of the body of the subject 1 based on the enlarged partial image 3 of substantially the same range regardless of the distance L1 between the optical imaging unit 30 and the X-ray detection unit 20.

Furthermore, in the present embodiment, as described above, the optical imaging control unit 32 is configured to display, on the display operation unit 41 as the enlarged partial image 3, the entire range of a predetermined region 7 preset based on the size of the X-ray detection unit 20 in real space for each imaging region. Thus, since the range of the predetermined region 7 is preset for each imaging region, the radiographer can easily adjust the body orientation and position of the subject 1 based on the enlarged partial image 3 by the range of the predetermined region 7 suitable for each imaging region.

In the present embodiment, as described above, the display operation unit 41 includes a fine adjustment enlargement operation unit 46 and a fine adjustment reduction operation unit 47 that receive an input operation for finely adjusting the enlargement ratio for the enlarged partial image 3, and the optical imaging control unit 32 is configured to generate a re-enlarged partial image 70 obtained by further enlarging the enlarged partial image 3 displayed on the display operation unit 41 or a reduced enlarged-partial image 71 obtained by reducing the enlarged partial image 3 displayed on the display unit based on an input operation to the fine adjustment enlargement operation unit 46 or the fine adjustment reduction operation unit 47, and to display the generated image on the display operation unit 41. Accordingly, by finely adjusting the enlarged partial image 3, the radiographer can easily fine-tune the body direction and position of the subject 1 based on a re-enlarged partial image 70 obtained by further enlarging the enlarged partial image 3 or a reduced enlarged-partial image 71 obtained by reducing the enlarged partial image 3.

In addition, in the present embodiment, as described above, the optical imaging control unit 32 is configured to set the center position 3a of the enlarged partial image 3 to a position substantially matching the position of the intersection 4, based on the relationship between the optical axis 5 of the optical imaging unit 30 and the irradiation axis 6 of the X-rays. Accordingly, the X-ray detection unit 20 and the subject 1 can be easily displayed in the center in the enlarged partial image 3, thereby making it possible to more effectively suppress the region of interest from not being displayed on the display operation unit 41 due to the region of interest of the subject 1 protruding outside the enlarged partial image 3.

### [Modifications]

It should be considered that the embodiments disclosed this time are illustrative in all respects and not restrictive. The scope of the present invention is shown by the claims rather than the above description of the embodiments, and includes all changes (modifications) within the meaning and scope equivalent to the claims.

For example, in the above-described embodiment, the optical imaging control unit including the microcomputer is configured to crop out the predetermined region from the optical image and generate the enlarged partial image that has been enlarged; however, the present invention is not limited to this. For example, the apparatus control unit that controls the X-ray imaging may be configured to crop out a predetermined region from the optical image and generate the enlarged partial image that has been enlarged.

Further, in the above embodiment, an example has been shown in which the optical imaging control unit is configured to set the center position of the enlarged partial image to a position substantially matching a position of an intersection of an X-ray detection unit in real space and an irradiation axis of X-rays irradiated from an X-ray irradiation unit, but the present invention is not limited thereto. For example, the optical imaging control unit may be configured such that the center position of the enlarged partial image is shifted from the center position of the optical image in a direction approaching the position of the intersection without shifting the center position of the enlarged partial image to a position substantially corresponding to the position of the intersection.

Furthermore, in the above-described embodiment, an example has been shown in which the optical imaging control unit is configured to have an optical axis substantially parallel to the irradiation axis of the X-rays irradiated from the X-ray irradiation unit, but the present invention is not limited to this. For example, the optical axis of the optical imaging control unit may be inclined with respect to an irradiation axis of X-rays irradiated from the X-ray irradiation unit.

Further, in the above embodiment, an example has been shown in which the optical imaging control unit sets the center position of the enlarged partial image to a position substantially matching a position of an intersection of the X-ray detection unit in real space and the irradiation axis of the X-rays irradiated from the X-ray irradiation unit, based on a distance between the X-ray irradiation unit and the X-ray detection unit, a distance between the irradiation axis of the X-rays and the optical axis of the optical imaging unit, and an angle of view of the optical imaging unit, but the present invention is not limited thereto. For example, the center position of the enlarged partial image may be set to a position substantially matching the position of the intersection from a combination of two of a distance between the X-ray irradiation unit and the X-ray detection unit, a distance between the irradiation axis of the X-rays and an optical axis of the optical imaging unit, and an angle of view of the optical imaging unit, or the center position of the enlarged partial image may be set to a position substantially matching the position of the intersection from a combination of four or more adding another parameter.

In the above embodiment, an example in which the optical imaging control unit is configured to set the center position of the enlarged partial image to a position substantially corresponding to the position of the intersection based on the above equation (1) is shown, but the present invention is not limited to this. For example, the optical imaging control unit may be configured to set the center position of the enlarged partial image to a position substantially matching the position of the intersection based on an equation other than the above equation (1).

Further, in the above embodiment, an example has been shown in which the optical imaging control unit is configured to display the range of the predetermined region in the optical image on the display operation unit as an enlarged partial image on the basis of an enlargement ratio preset for each imaging region, but the present invention is not limited thereto. For example, the optical imaging control unit may be configured to cause the display operation unit to display the range of the predetermined region in the optical image as the enlarged partial image based on each operative method or each distance between the X-ray irradiation unit and the X-ray detection unit, or may be configured to cause the display operation unit to display the range of the predetermined region in the optical image as the enlarged partial image based on an enlargement ratio set in advance based on a combination of at least two of each imaging region, each operative method, and each distance between the X-ray irradiation unit and the X-ray detection unit.

In the above-described embodiment, an example is shown in which the optical imaging control unit is configured to display on the display operation unit as the enlarged partial image the entire range of a predetermined region preset for each imaging region based on a size of the X-ray detection unit in real space, but the present invention is not limited to this. For example, the optical imaging control unit may be configured to display, on the display operation unit as an enlarged partial image, the entire range of a predetermined region preset for each operative method based on a size of the X-ray detection unit in real space, or may be configured to display, on the display operation unit as an enlarged partial image, the entire range of a predetermined region preset for each combination of an imaging region and an operative method based on a size of the X-ray detection unit in real space.

Furthermore, in the above-described embodiment, an example is shown in which the optical imaging control unit adjusts the enlargement ratio for generating the enlarged partial image based on the above equation (2), but the present invention is not limited to this. For example, the optical imaging control unit may be configured to adjust the enlargement ratio for generating the enlarged partial image based on an equation other than the equation (2) described above.

### [Aspects]

It will be understood by those skilled in the art that the illustrative embodiments described above are specific examples of the following aspects.

(Item 1) An X-ray imaging apparatus including: an X-ray irradiation unit including an X-ray tube; an X-ray detection unit that detects X-rays irradiated from the X-ray irradiation unit and transmitted through a subject; an optical imaging unit that has an optical axis offset with respect to an irradiation axis of the X-rays irradiated from the X-ray irradiation unit and captures an optical image of the subject; a control unit that crops out a predetermined region from the optical image and generates an enlarged partial image that has been enlarged; and a display unit that displays the enlarged partial image generated by the control unit, wherein the control unit is configured to, based on a relationship between the optical axis of the optical imaging unit and the irradiation axis of the X-rays, set a center position of the enlarged partial image to a position shifted from a center position of the optical image in a direction approaching a position of an intersection of the X-ray detection unit in real space and the irradiation axis of the X-rays irradiated from the X-ray irradiation unit.

### (Item 2)

The X-ray imaging apparatus according to item 1, wherein the control unit is configured to shift the center position of the enlarged partial image in a direction approaching a position of the intersection based on a distance between the optical imaging unit and the X-ray detection unit, a distance between the irradiation axis of the X-rays and the optical axis of the optical imaging unit, and an angle of view of the optical imaging unit.

### (Item 3)

The X-ray imaging apparatus according to item 1 or 2, wherein the optical imaging unit is configured to have an optical axis substantially parallel to the irradiation axis of the X-rays irradiated from the X-ray irradiation unit.

### (Item 4)

The X-ray imaging apparatus according to any one of items 1 to 3, wherein the control unit is configured to display a range of the predetermined region in the optical image on the display unit as the enlarged partial image based on an enlargement ratio set in advance.

### (Item 5)

The X-ray imaging apparatus according to item 4, wherein the control unit is configured to display a range of the predetermined region in the optical image on the display unit as the enlarged partial image based on the enlargement ratio set in advance based on at least one of each imaging region, each operative method, and each distance between the X-ray irradiation unit and the X-ray detection unit.

### (Item 6)

The X-ray imaging apparatus according to any one of items 1 to 5, wherein the control unit is configured to adjust an enlargement ratio for generating the enlarged partial image, thereby displaying an entire range of the predetermined region set in advance based on a size of the X-ray detection unit in real space on the display unit as the enlarged partial image.

### (Item 7)

The X-ray imaging apparatus according to item 6, wherein the control unit is configured to cause the display unit to display as the enlarged partial image the entire range of the predetermined region preset based on the size of the X-ray detection unit in real space for at least one of each imaging region and each operative method.

### (Item 8)

The X-ray imaging apparatus according to any one of items 1 to 7, wherein the display unit includes an operation unit that accepts an input operation for fine adjustment of an enlargement ratio with respect to the enlarged partial image, and the control unit is configured to generate a re-enlarged partial image obtained by further enlarging the enlarged partial image displayed on the display unit or a reduced enlarged-partial image obtained by reducing the enlarged partial image displayed on the display unit, based on the input operation to the operation unit, and to cause the display unit to display the generated image.

### (Item 9)

The X-ray imaging apparatus according to any one of items 1 to 8, wherein the control unit is configured to set a center position of the enlarged partial image to a position substantially coinciding with a position of the intersection based on a relationship between the optical axis of the optical imaging unit and the irradiation axis of the X-rays.

### Description of Symbols

1 Subject
2 Optical image
2a Center position of optical image
3 Enlarged partial image
3a Center position of enlarged partial image
4 Intersection of an X-ray detection unit in real space and an irradiation axis of X-rays
irradiated from an X-ray irradiation unit
5 Optical axis
6 X-ray irradiation axis
7 Predetermined region
10 X-ray irradiation unit
11 X-ray tube 20 X-ray detection unit
30 Optical imaging unit
32 Optical imaging control unit (control unit)
41 Display operation unit (display unit, operation unit)
46 Fine adjustment enlargement operation unit (operation unit)
47 Fine adjustment reduction operation unit (operation unit)
70 Re-enlarged partial image
71 Reduced enlarged-partial image
100 X-ray imaging apparatus
L1 Distance between an optical imaging unit and an X-ray detection unit
D1 Distance between an X-ray irradiation axis and an optical axis of an optical imaging unit *θ* Angle of view of an optical imaging unit

## Claims

1. An X-ray imaging apparatus comprising: an X-ray irradiation unit including an X-ray tube; an X-ray detection unit that detects X-rays irradiated from the X-ray irradiation unit and transmitted through a subject; an optical imaging unit that has an optical axis offset with respect to an irradiation axis of the X-rays irradiated from the X-ray irradiation unit and captures an optical image of the subject; a control unit that crops out a predetermined region from the optical image and generates an enlarged partial image that has been enlarged; and a display unit that displays the enlarged partial image generated by the control unit, wherein the control unit is configured to, based on a relationship between the optical axis of the optical imaging unit and the irradiation axis of the X-rays, set a center position of the enlarged partial image to a position shifted from a center position of the optical image in a direction approaching a position of an intersection of the X-ray detection unit in real space and the irradiation axis of the X-rays irradiated from the X-ray irradiation unit.

2. The X-ray imaging apparatus according to claim 1, wherein the control unit is configured to set the center position of the enlarged partial image to a position shifted in a direction approaching a position of the intersection based on a distance between the optical imaging unit and the X-ray detection unit, a distance between the irradiation axis of the X-rays and the optical axis of the optical imaging unit, and an angle of view of the optical imaging unit.

3. The X-ray imaging apparatus according to claim 1, wherein the optical imaging unit is configured to have an optical axis substantially parallel to the irradiation axis of the X-rays irradiated from the X-ray irradiation unit.

4. The X-ray imaging apparatus according to claim 1, wherein the control unit is configured to cause the display unit to display a range of the predetermined region in the optical image as the enlarged partial image based on a preset enlargement ratio.

5. The X-ray imaging apparatus according to claim 4, wherein the control unit is configured to cause the display unit to display a range of the predetermined region in the optical image as the enlarged partial image based on the preset enlargement ratio based on at least one of each imaging region, each operative method, and each distance between the X-ray irradiation unit and the X-ray detection unit.

6. The X-ray imaging apparatus according to claim 1, wherein the control unit is configured to adjust an enlargement ratio for generating the enlarged partial image, thereby displaying an entire range of the predetermined region set in advance based on a size of the X-ray detection unit in real space on the display unit as the enlarged partial image.

7. The X-ray imaging apparatus according to claim 6, wherein the control unit is configured to cause the display unit to display, as the enlarged partial image, the entire range of the predetermined region preset based on the size of the X-ray detection unit in real space for at least one of each imaging region and each operative method.

8. The X-ray imaging apparatus according to claim 1, wherein the display unit includes an operation unit that accepts an input operation for fine adjustment of an enlargement ratio with respect to the enlarged partial image, and the control unit is configured to generate a re-enlarged partial image obtained by further enlarging the enlarged partial image displayed on the display unit or a reduced enlarged-partial image obtained by reducing the enlarged partial image displayed on the display unit, based on the input operation to the operation unit, and to display the generated image on the display unit.

9. The X-ray imaging apparatus according to claim 1, wherein the control unit is configured to set the center position of the enlarged partial image to a position substantially matching a position of the intersection based on a relationship between the optical axis of the optical imaging unit and the irradiation axis of the X-rays.
